(19) Europäisches Patentamt
European Patent Office
Office européen des brevets

(11) Publication number: 0 283 194 B1

(12) # EUROPEAN PATENT SPECIFICATION

(45) Date of publication of patent specification: **17.06.92**

(51) Int. Cl.5: **C07C 51/10**, C07C 63/06, C07C 63/04, C07C 63/70, B01J 31/28

(21) Application number: **88302016.6**

(22) Date of filing: **09.03.88**

(54) **Process for producing carboxylic acids.**

(30) Priority: **14.03.87 JP 59686/87**
**13.02.88 JP 31140/88**

(43) Date of publication of application:
**21.09.88 Bulletin 88/38**

(45) Publication of the grant of the patent:
**17.06.92 Bulletin 92/25**

(84) Designated Contracting States:
**CH DE FR GB IT LI**

(56) References cited:
**EP-A- 0 086 281**
**EP-A- 0 239 145**
**EP-A- 0 255 794**
**US-A- 2 565 461**
**US-A- 3 988 358**

**BEILSTEINS HANDBUCH DER ORGANISCHEN CHEMIE, 4th edition, vol. 9, 2nd supplement, 1949, Springer Verlag Berlin**

(73) Proprietor: **NIHON NOHYAKU CO., LTD.**
**1-2-5, Nihonbashi**
**Chuo-ku Tokyo(JP)**

(72) Inventor: **Suto, Keiji**
**7-20, Nigawayurinocho**
**Nishinomiya-shi(JP)**
Inventor: **Nakasa, Koji**
**6-7-301, Sagisu-3-chome**
**Fukushima-ku Osaka(JP)**
Inventor: **Kudo, Masaaki**
**11-1-701, Ebatacho**
**Kadoma-shi(JP)**
Inventor: **Yamamoto, Moriharu**
**11-1, Nishiokamoto-6-chome**
**Higashinada-ku Kobe(JP)**

(74) Representative: **Votier, Sidney David et al**
**CARPMAELS & RANSFORD 43, Bloomsbury Square**
**London WC1A 2RA(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for producing an aromatic or heterocyclic carboxylic acid and more particularly it relates to a process for producing a carboxylic acid represented by the general formula (I):

$R^1 \cdot COOH$ (I)

(wherein $R^1$ is a substituted or unsubstituted, aromatic or heterocyclic hydrocarbon group), which comprises reacting an organic chloride represented by the general formula (II):

$R^1 \cdot Cl$ (II)

(wherein $R^1$ has the same meaning as defined above) with carbon monoxide at a reaction temperature of 150° to 300°C in the presence of a carbonylation catalyst composed of a palladium compound and a phosphine compound represented by the general formula (III):

$(R)_2P - X - P(R)_2$ (III)

(wherein R is an alkyl group or a substituted or unsubstituted phenyl group, and X is an alkylene group having 1 to 6 carbon atoms,

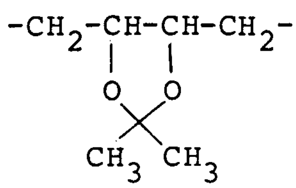
,
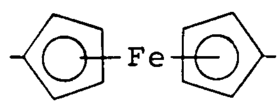
,

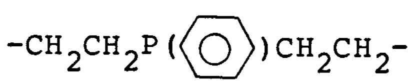

or a binaphthyl group), and an inorganic base or an organic base/water.

The carboxylic acids of the general formula (I) obtained by this invention are useful not only as medicines and agricultural chemicals but also as various industrial materials, and the present invention provides a novel process for producing these carboxylic acids.

RELATED ART

As a conventional process for producing a carboxylic acid, there is suggested in U.S. Patent 3988358 a process in which carboxylic acid is produced by reacting an aromatic halide with carbon monoxide and water in the presence of a palladium/phosphine catalyst. However, this process is not exemplified by Working Examples in U.S. Patent 3988358.

In J.O.C. 1981, 46, 4614-4617, a process for producing a carboxylic acid from an aryl halide is described, but although the desired carboxylic acid can be obtained from a bromide, it cannot be obtained from a chloride because no reaction proceeds.

Jap. Pat. Appln. Kokai (Laid-Open) No. 61-233648 discloses a process in which a poly(arylcarboxylic acid) is produced under irradiation with light by using cobalt carbonyl as a catalyst. However, since this process is practised under irradiation with light, it requires facilities for the irradiation and its industrialization entailes great cost. Moreover, this process uses cobalt carbonyl and hence is disadvantageous also from the viewpoint of toxicity. As a process in which calbonylation of an aromatic chloride is carried out, Jap. Pat. Appln. Kokai (Laid-Open) No. 61-293950 discloses a process for producing a carboxylic acid using a chloro- or bromo-allenecarbonylchromium compound, but this process is disadvantageous in that a tricarbonylchromium complex as reaction substrate should be produced.

As described above, the reactions of aromatic iodides or bromides with carbon monoxide are known, but there is not known any process in which an aromatic or heterocyclic carboxylic acid is synthesized by the reaction of an aromatic or heterocyclic chloride with carbon monoxide.

SUMMARY OF THE INVENTION

In consideration of such conditions, the present inventors earnestly investigated the reactions of aromatic or heterocyclic chlorides with carbon monoxide and consequently established a process for producing an aromatic or heterocyclic carboxylic acid in a high yield, whereby the present invention has been accomplished.

The process for producing a carboxylic acid of this invention can be schematically represented, for example, as follows:

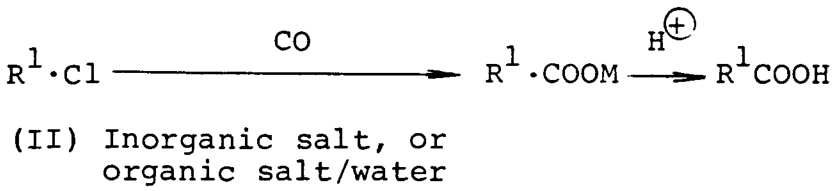


wherein $R^1$ has the same meaning as defined above, and M is a base residue.

That is to say, an organic chloride of the general formula (II) is reacted with carbon monoxide at a reaction temperature of 150° to 300°C in the presence or absence of a solvent in the presence of a carbonylation catalyst composed of a palladium compound and a phosphine compound of the general formula (III):

$(R)_2P - X - P(R)_2$ (III)

(wherein R is an alkyl group or a substituted or unsubstituted phenyl group, and X is an alkylene group having 1 to 6 carbon atoms,

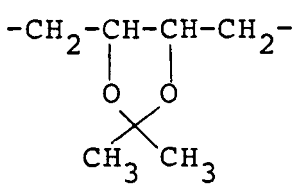,
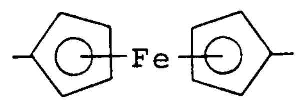,

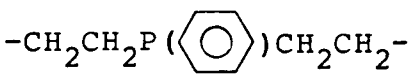

or a binaphthyl group), and an inorganic base or an organic base/water, whereby a desired carboxylic acid of the general formula (I) can be obtained.

DETAILED DESCRIPTION OF PREFERRED EMBODIMENTS

When an inorganic salt is used in the reaction of this invention, addition of water is not necessary because water generated by the reaction is present in the reaction system, but when an organic base is used therein, use of water is essential and the using amount is generally equimolar with or in excess of the organic chloride of the general formula (II).

The organic chloride of the general formula (II) in this invention may be any one so long as it has at least one chlorine atom on its ring of a substituted or unsubstituted, aromatic or heterocyclic hydrocarbon. It includes also fused ring hydrocarbons and fused heterocyclic ring hydrocarbons. Typical examples of said organic chloride includes, for example, aromatic organic chlorides such as chlorobenzene, dichlorobenzene, trichlorobenzene, tetrachlorobenzene, pentachlorobenzene, hexachlorobenzene, chlorofluorobenzene, chlorodifluorobenzene, chlorotrifluorobenzene, chlorotetrafluorobenzene, chloropentafluorobenzene, trifluoromethylchlorobenzene, chlorotoluene, dichlorotoluene, trichlorotoluene, chloroxylene, dichloroxylene, trichloroxylene, chlorophenol, chloroanisole, chloronitrobenzene, dichloronitrobenzene, chlorocyanobenzene, chlorophenylacetic acid esters, N-acetylchloroaniline, chloroacetophenone, chlorobenzophenone,

chloromethylthiobenzene, chlorobenzoic acid esters, chlorodiphenyl ether, dichlorodiphenyl ether, dichlorobenzophenone, dichlorodiphenyl sulfone, dichlorodiphenylmethane, dichlorodiphenyl, chloronaphthalene, chloromethylnaphthalene, chloroanthraquinone, and the like; and heterocyclic organic chlorides such as chlorothiophene, chlorofuran, chloroindole, chloropyridine, dichloropyridine, chloropicoline, chloroquinoline, chloroquinoxaline, dichloroquinoxaline, and the like. The organic chloride may be used in a predetermined amount only as a reactant, or it may be added in excess and used both as a reactant and a solvent.

The palladium compound as carbonylation catalyst in this invention is used in combination with a phosphine compound. The palladium compound includes, for example, metallic palladium, palladium carbon, palladium alumina, palladium chloride, pallaidum bromide, palladium acetate, dichlorobiscyanophenylpalladium, dichlorobistriphenylphosphine palladium, tetrakistriphenylphosphine palladium, etc. The phosphine compound of the general formula (III) includes, for example, bis-(dialkylphosphino)alkanes such as 1,1-bis(dimethylphosphino)methane, 1,1-bis(diethylphosphino)methane, 1,2-bis(dimethylphosphino)ethane, 1,2-bis(diethylphosphino)ethane, 1,3-bis(dimethylphosphino)propane, 1,4-bis(dimethylphosphino)butane, and the like, bis(diphenylphosphino)alkanes such as 1,1-bis-(diphenylphosphino)methane, 1,2-bis(diphenylphosphino)ethane, 1,3-bis(diphenylphosphino)propane, 1,4-bis(diphenylphosphino)butane, 1,5-bis(diphenylphosphino)pentane, 1,6-bis(diphenylphosphino)hexane, 2,3-O-isopropylidene-2,3-dihydroxy-1,4-bisdiphenylphosphinobutane, and the like, bisdiphenylphosphinoferrocene, bisdiphenylphosphinobinaphthyl, 1,2-bis(diphenylphosphino)benzene, 1,1-bis(dibenzophosphoryl)-methane, 1,2-bis(dibenzophosphoryl)ethane, 1,3-bis(dibenzophosphoryl)propane, 1,4-bis-(dibenzophosphoryl)butane, 1,5-bis(dibenzophosphoryl)pentane, etc.

The adding amount of the phosphine compound is generally 0.01 to 10,000 moles, preferably 0.1 to 100 moles per mole of the palladium compound.

In this invention, the palladium compound and the phosphine compound of the general formula (III) are used in combination and may be used in the reaction system either individually or in the form of a previously prepared complex.

Although not critical, the total amount of the palladium compound and the phosphine compound added to the reaction system is generally 0.0001 to 0.5 mole, preferably 0.001 to 0.1 mole per mole of the organic chloride of the general formula (II).

The inorganic base used in this invention includes sodium carbonate, potassium carbonate, sodium hydroxide, potassium hydroxide, etc. The organic base used therein includes triethylamine, tributylamine, diisopropylethylamine, triisooctylamine, pyridine, N-methylpyrrolidine, N-methylmorpholine, N-ethylmorpholine, etc.

Although the base is used preferably in an amount required for neutralization of hydrogen chloride generated, the using amount may, of course, be smaller or larger than this amount.

When an organic base is used, use of water is essential and the using amount of water is generally 0.1 to 100 moles per mole of the organic chloride.

The reaction in this invention can be carried out in the presence or absence of a solvent, and any solvent can be used so long as it does not inhibit the reaction seriously. As such a solvent, there may be exemplified, for example, organic solvents such as hexane, benzene, ether, tetrahydrofuran, acetonitrile, dimethylformamide, hexamethylphosphotriamide, acetone, etc.

The reaction in this invention is carried out at atmospheric pressure or under pressure. The pressure of carbon monoxide is properly selected in the range of 1 to 200 atmospheres, preferably 1 to 50 atmospheres.

The reaction temperature in this invention is 150° to 300°C, preferably 150° to 250°C.

As a reactor used in this invention, a conventional one may be used. When the reaction is carried out under pressure, any reactor may be used so long as it can withstand the reaction pressure, and usually a reactor made of metal or glass is used.

Although varied depending on the amounts of the reactions and the reaction temperature, the reaction time is generally selected in the range of several minutes to 48 hours.

In this invention, when the organic chloride of the general formula (II) is an organic polychlorinated compound, the chlorine atoms on the ring can be selectively made into acid in turn.

A desired compound can be obtained by treating the reaction mixture by a conventional method after completion of the reaction.

Examples of this invention are described below but are not by way of limitation but by way of illustration.

Example 1

Production of benzoic acid

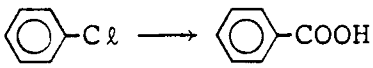

In an autoclave made of metal were placed 11.2 g of chlorobenzene, 17.5 mg of palladium chloride, 427 mg of bisdiphenylphosphinobutane and 3.1 g of potassium carbonate. The air in the autoclave was replaced with carbon monoxide introduced thereinto in several times, after which carbon monoxide was further introduced to its pressure therein to 50 kg/$cm^2$. The reaction was carried out with stirring for 3 hours on a salt bath at a bath temperature of 210°C. After completion of the reaction, the reaction mixture was cooled to room temperature and water was added. The aqueous layer was separated, after which acid was added thereto and the deposited crystals were collected by filtration and dried to obtain 0.97 g of the desired compound benzoic acid.

Melting point 122° - 123°C

Example 2

Production of ortho-chlorobenzoic acid

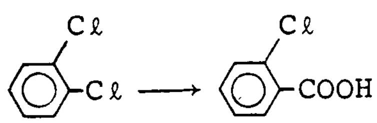

The procedure of Example 1 was repeated, except that 14.7 g of ortho-dichlorobenzene was used in place of 11.2 g of chlorobenzene. Thus, 0.77 g of the desired compound ortho-chlorobenzoic acid was obtained.

Melting point 138° - 140°C

Example 3

Production of ortho-methylbenzoic acid

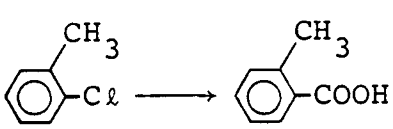

The procedure of Example 1 was repeated, except that 12.6 g of ortho-chlorotoluene was used in place of 11.2 g of chlorobenzene. Thus, 2.35 g of the desired compound ortho-methylbenzoic acid was obtained.

Melting point 103° - 105°C

Example 4

Production of para-chlorobenzoic acid

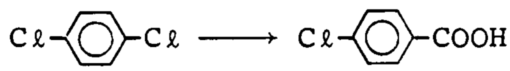

In an autoclave made of metal were placed 2.78 g of para-dichlorobenzene, 17.5 mg of palladium chloride, 427 mg of bisdiphenylphosphinobutane, 3.1 g of potassium carbontae and 30 ml of acetonitrile. The air in the autoclave was replaced with carbon monoxide introduced thereinto in several times, after which carbon monoxide was further introduced to adjust its pressure therein to 50 kg/cm$^2$. The reaction was carried out with stirring for 3 hours on a salt bath at a bath temperature of 250 °C. After completion of the reaction, the reaction mixture was cooled to room temperature and water was added. The aqueous layer was separated and then acid was added thereto, followed by extraction from the aqueous layer with ether. The ether layer was dried and then concentrated to obtain 0.6 g of para-chlorobenzoic acid.

Melting point 239° - 241 °C

Example 5

Production of 2,3-dichlorobenzoic acid

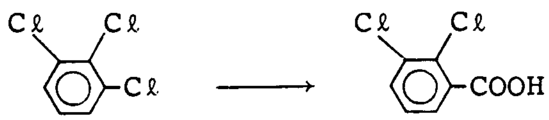

The procedure of Example 4 was repeated, except that 18.1 g of 1,2,3-trichlorobenzene was used in place of 2.78 g of para-dichlorobenzene. Thus, 1.43 g of the desired compound 2,3-dichlorobenzoic acid was obtained.

Melting point 167° - 169 °C

Example 6

Production of ortho-trifluoromethylbenzoic acid

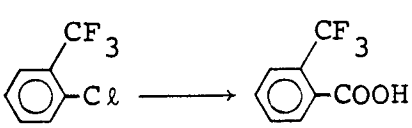

In an autoclave made of metal were placed 18 g of ortho-chlorobenzotrifluoride, 3.5 mg of palladium chloride, 170 mg of bisdiphenylphosphinobutane and 3.1 g of potassium carbonate. The air in the autoclave was replaced with carbon monoxide introduced thereinto in several times, after which carbon monoxide was further introduced to adjust its pressure therein to 30 kg/cm$^2$. The reaction was carried out with stirring on a salt bath at a bath temperature of 240 °C. After completion of the reaction, the reaction mixture was cooled to room temperature and water was added. The aqueous layer was separated and then acid was added thereto, followed by extraction from the aqueous layer. The ether layer was dried and then concentrated to obtain 3.9 g of ortho-trifluoromethylbenzoic acid.

Melting point 109° - 113 °C

Example 7

Production of ortho-trifluorobenzoic acid

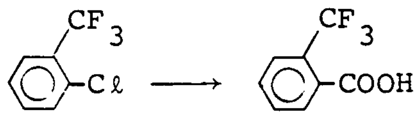

In an autoclave made of metal were placed 18 g of ortho-chlorobenzotrifluoride, 3.5 mg of palladium chloride, 170 mg of bisdiphenylphosphinobutane and 23 g of sodium carbonate. The air in the autoclave was replaced with carbon monoxide introduced thereinto in several times, after which carbon monoxide was further introduced to adjust its pressure therein to 30 kg/cm$^2$. The reaction was carried out with stirring for 5 hours on a salt bath at a bath temperature of 210 °C. After completion of the reaction, the reaction mixture was cooled to room temperature and water was added. The aqueous layer was separated and then acid was added thereto, followed by extraction from the aqueous layer with ether. The ether layer was dried and then concentrated to obtain 2.8 g of ortho-trifluoromethylbenzoic acid.

Example 8

Production of meta-methylbenzoic acid

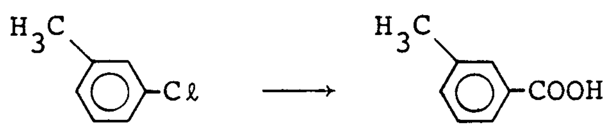

In an autoclave made of metal were placed 2.53 g of meta-chlorotoluene, 35.5 mg of palladium chloride, 426 mg of bisdiphenylphosphinobutane and 3.04 g of potassium carbonate. The air in the autoclave was replaced with carbon monoxide introduced thereinto in several times, after which carbon monoxide was further introduced to adjust its pressure therein to 50 kg/cm$^2$. The reaction was carried out with stirring for 3 hours on a salt bath at a bath temperature of 210 °C. After completion of the reaction, the reaction mixture was cooled to room temperature and water was added. The aqueous layer was separated, after which acid was added thereto and the deposited crystals were collected by filtration and dried. Thus, 1.10 g of the desired compound meta-methylbenzoic acid was obtained.

Melting point 107 ° - 110 °C

Example 9

The procedure of Example 8 was repeated, except that each chloride and each base listed in Table 1 were used in place of the chloride and the base, respectively, used in Example 8.

Table 1

| Example | Chloride | | Base | | Acid | |
|---|---|---|---|---|---|---|
| 9-1 | p-Chlorotoluene | 1.27 g | Potassium carbonate | 1.52 g | p-Methylbenzoic acid | 0.67 g |
| 9-2 | 1,2-Dichhorobenzene | 2.92 g | Sodium carbonate | 2.33 g | o-Chlorobenzoic acid | 2.19 g |
| 9-3 | 1,3-Dichlorobenzene | 2.92 g | Sodium carbonate | 2.33 g | m-Chlorobenzoic acid | 0.78 g |
| 9-4 | 1,2,4,5-Tetra-chlrobenzene | 21.5 g | Sodium carbonate | 11.66 g | 2,4,5-Trichloro-benzoic acid | 1.38 g |
| 9-5 | 2,5-Dichloropara-xylene | 3.50 g | Potassium carbonate | 3.04 g | 4-Chloro-2,5-dimethyl-benzoic acid | 2.16 g |
| 9-6 | 4-Chlorocyano-benzene | 2.75 g | Sodium carbonate | 2.75 g | p-Cyanobenzoic acid | 1.78 g |

Example 10

Production of para-methoxybenzoic acid

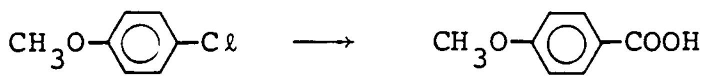

In an autoclave made of metal were placed 2.85 g of para-methoxychlorobenzene, 35.5 mg of palladium chloride, 426 mg of bisdiphenylphosphinobutane, 3.04 g of potassium carbonate and 30 mg of benzene. The air in the autoclave was replaced with carbon monoxide introduced thereinto in several times, after which carbon monoxide was further introduced to adjust its pressure therein to 40 kg/cm$^2$. The reaction was carried out with stirring for 3 hours on a salt bath at a bath temperature of 220 ° C. After completion of the reaction, the reaction mixture was cooled to room temperature and water was added. The benzene layer and the aqueous layer was separated from each other, after which acid was added to the aqueous layer, and the deposited crystals were collected by filtration and dried to obtain 1.57 g of the desired compound.

Melting point 183.5 ° - 184 ° C

Example 11

Production of thiophene-2-carboxylic acid

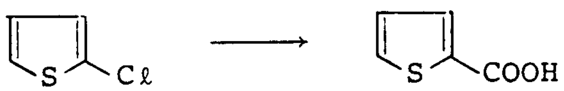

In an autoclave made of metal were placed 7.1 g of 2-chlorothiophene, 17.5 mg of palladium chloride, 426 mg of bisdiphenylphosphinobutane, 3.0 g of potassium carbonate, and 7.8 g of benzene. The air in the autoclave was replaced with carbon monoxide introduced in several times, after which carbon monoxide was further introduced to adjust its pressure therein to 30 kg/cm$^2$. The reaction was carried out with stirring for 3 hours on a salt bath at a bath temperature of 225 ° C. After completion of the reaction, the reaction mixture was cooled to room temperature and water was added. The benzene layer and the aqueous layer were separated from each other, after which acid was added to the aqueous layer and the deposited crystals were collected by filtration and dried to obtain 0.35 g of the desired compound thiophene-2-carboxylic acid.

Melting point 127 ° - 134 ° C

## Claims

**1.** A process for producing a carboxylic acid which comprises reacting an organic chloride having at least one chlorine atom on its ring of a substituted or unsubstituted, aromatic or heterocyclic hydrocarbon with carbon monoxide in the presence of an inorganic base at a reaction temperature of 150 ° to 300 ° C by using as catalysts a palladium compound and a phosphine compound represented by the general formula (III):

$(R)_2P - X - P(R)_2$ (III)

wherein R is an alkyl group or a substituted or unsubstituted phenyl group, and X is an alkylene group having 1 to 6 carbon atoms,

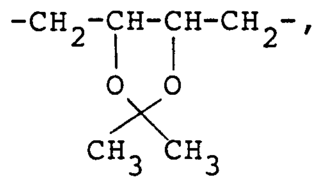 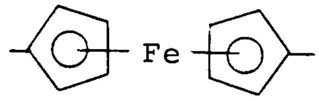 ,

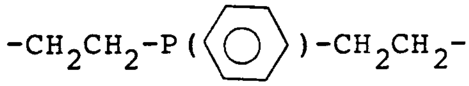

or a binaphthyl group.

2. A process for producing a carboxylic acid according to Claim 1, wherein the palladium compound is metallic palladium, metallic palladium supported on a solid, or a zerovalent, divalent or tetravalent palladium complex.

3. A process for producing a carboxylic acid according to Claim 2, wherein the palladium compound is palledium carbon, palladium chloride or palladium acetate.

4. A process for producing a carboxylic acid according to Claim 3, wherein the phosphine compound is a bis(diphenylphosphino)alkane.

5. A process for producing a carboxylic acid according to Claim 4, wherein the base is sodium carbonate or potassium carbonate.

6. A process for producing a carboxylic acid according to Claim 5, wherein the pressure of carbon monoxide is 1 to 200 atmospheres.

7. A process for producing a carboxylic acid which comprises reacting an organic chloride having at least one chlorine atom on its ring of a substituted or unsubstituted, aromatic or heterocyclic hydrocarbon with carbon monoxide and water in the presence of an organic base at a reaction temperature of 150° to 300°C by using as catalysts a palladium compound and a phosphine compound represented by the general formula (III):

$(R)_2P - X - P(R)_2$ (III)

wherein R is an alkyl group or a substituted or unsubstituted phenyl group, and X is an alkylene group having 1 to 6 carbon atoms,

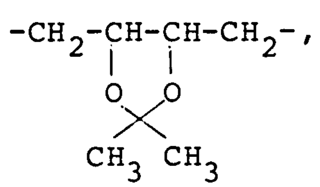 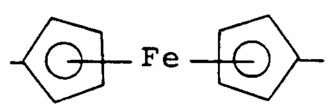 ,

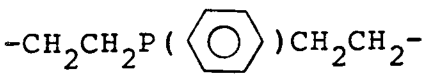

or a binaphthyl group.

8. A process for producing a carboxylic acid according to Claim 7, wherein the palladium compound is a metallic palladium, metallic palladium supported on a solid, or a zerovalent, divalent or tetravalent palladium complex.

9. A process for producing a carboxylic acid according to Claim 8, wherein the palladium compound is

palladium carbon, palladium chloride or palladium acetate.

**10.** A process for producing a carboxylic acid according to Claim 8, wherein the phosphine compound is a bis(diphenylphosphino)alkane.

**11.** A process for producing a carboxylic acid according to Claim 10, wherein the base is tributylamine or N-ethylmorpholine.

**12.** A process for producing a carboxylic acid according to Claim 11, wherein the pressure of carbon monoxide is 1 to 200 atmospheres.

**Revendications**

**1.** Procédé de préparation d'un acide carboxylique, selon lequel on fait réagir un chlorure organique comportant au moins un atome de chlore sur son cycle dérivé d'un hydrocarbure aromatique ou hétérocyclique, substitué ou non substitué, avec du monoxyde de carbone en présence d'une base inorganique, à une température réactionnelle de 150° à 300° C, en employant comme catalyseurs, un composé du palladium et un composé de phosphine de formule générale (III):

$(R)_2P\text{-}X\text{-}P(R)_2$ (III)

dans laquelle R représente un groupe alkyle, ou un groupe phényle substitué ou non substitué, et X représente un groupe alkylène comportant de 1 à 6 atomes de carbone,

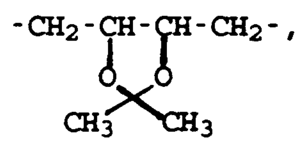

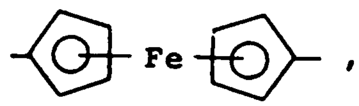

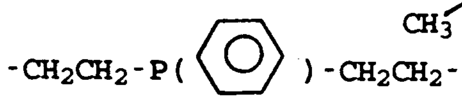

ou un groupe binaphtyle.

**2.** Procédé de préparation d'un acide carboxylique selon la revendication 1, dans lequel le compose du palladium, est du palladium métallique, du palladium métallique supporté par un solide, ou un complexe du palladium zérovalent, divalent ou tétravalent.

**3.** Procédé de préparation d'un acide carboxylique selon la revendication 2, dans lequel le composé du palladium est du palladium sur carbone, du chlorure de palladium ou de l'acétate de palladium.

**4.** Procédé de préparation d'un acide carboxylique selon la revendication 3, dans lequel le composé de phosphine, est un bis(diphénylphosphino)alcane.

**5.** Procédé de préparation d'un acide carboxylique selon la revendication 4, dans lequel la base est le carbonate de sodium ou le carbonate de potassium.

**6.** Procédé de préparation d'un acide carboxylique selon la revendication 5, dans lequel la pression du monoxyde de carbone, est de 1 à 200 atmosphères.

**7.** Procédé de préparation d'un acide carboxylique, selon lequel on fait réagir un chlorure organique comportant au moins un atome de chlore sur son cycle dérivé d'un hydrocarbure aromatique ou hétérocyclique, substitué ou non substitué, avec du monoxyde de carbone et de l'eau en présence d'une base organique, et à une température réactionnelle de 150° à 300° C, en employant comme catalyseurs, un composé du palladium et un composé de phosphine de formule générale (III):

$(R)_2P\text{-}X\text{-}P(R)_2$ (III)

dans laquelle R représente un groupe alkyle, ou un groupe phényle substitué ou non substitué, et X représente un groupe alkylène comportant de 1 à 6

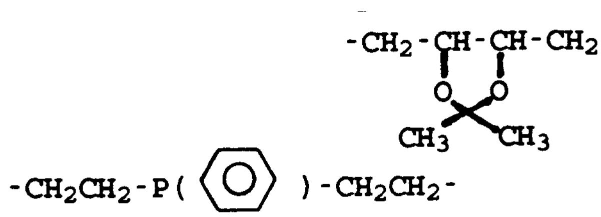,

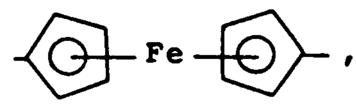,

atomes de carbone, ou un groupe binaphtyle.

8. Procédé de préparation d'un acide carboxylique selon la revendication 7, dans lequel le composant dérivé du palladium, est du palladium métallique, du palladium métallique supporté par un solide, ou un complexe du palladium zérovalent, divalent ou tétravalent.

9. Procédé de préparation d'un acide carboxylique selon la revendication 8, dans lequel le composé du palladium, est du palladium sur carbone, du chlorure de palladium ou de l'acétate de palladium.

10. Procédé de préparation d'un acide carboxylique selon la revendication 8, dans lequel le composé de phosphine, est un bis(diphénylphosphino)alcane.

11. Procédé de préparation d'un acide carboxylique selon la revendication 10, dans lequel la base est la tributylamine ou la N-éthylmorpholine.

12. Procédé de préparation d'un acide carboxylique selon la revendication 11, dans lequel la pression du monoxyde de carbone, est de 1 à 200 atmosphères.

## Patentansprüche

1. Verfahren zur Herstellung einer Carbonsäure, das die Umsetzung eines organischen Chlorids mit mindestens einem Chloratom an seinem Ring aus einem substituierten oder unsubstituierten aromatischen oder heterozyklischen Kohlenwasserstoff mit Kohlenmonoxid in Gegenwart einer anorganischen Base bei einer Reaktionstemperatur von 150° bis 300° C unter Verwendung einer Palladiumverbindung und einer durch die allgemeine Formel III dargestellten Phosphinverbindung als Katalysatoren umfaßt:

$(R)_2P - X - P(R)_2$ (III)

worin R eine Alkylgruppe oder eine substituierte oder unsubstituierte Phenylgruppe bedeutet und X eine Alkylengruppe mit 1 bis 6 Kohlenstoffatomen,

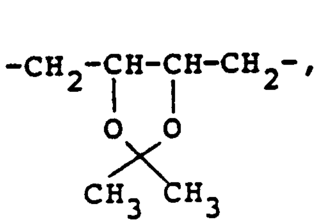, 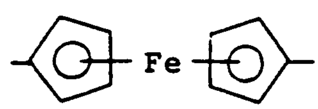, 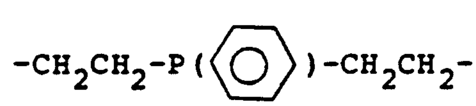

oder eine Binaphtylgruppe bedeutet.

2. Verfahren zur Herstellung einer Carbonsäure gemäß Anspruch 1, worin die Palladiumverbindung metallisches Palladium, metallisches Palladium auf einem Feststoffträger, oder ein nullvalenter, divalenter oder ein tetravalenter Palladiumkomplex ist.

**3.** Verfahren zur Herstellung einer Cabonsäure gemäß Anspruch 2, worin die Palladiumverbindung Palladium- Kohlenstoff, Palladiumchlorid oder Palladiumacetat ist.

**4.** Verfahren zur Herstellung einer Carbonsäure gemäß Anspruch 3, worin die Phosphinverbindung ein Bis(diphenylphosphino)alkan ist.

**5.** Verfahren zur Herstellung einer Carbonsäure gemäß Anspruch 4, worin die Base Natriumcarbonat oder Kaliumcarbonat ist.

**6.** Verfahren zur Herstellung einer Carbonsäure gemäß Anspruch 5, worin der Kohlenmonoxiddruck 1 bis 200 Atmosphären beträgt.

**7.** Verfahren zur Herstellung einer Carbonsäure, das die Umsetzung eines organischen Chlorids mit mindestens einem Chloratom an seinem Ring aus einem substituierten oder unsubstituierten aromatischen oder heterozyklischen Kohlenwasserstoff mit Kohlenmonoxid und Wasser in Anwesenheit einer organischen Base bei einer Reaktionstemperatur von 150° bis 300° C unter Verwendung einer Palladiumverbindung und einer durch die allgemeine Formel III dargestellten Phosphinverbindung als Katalysatoren umfaßt:

$(R)_2P - x - P(R)_2$ (III)

worin R eine Alkylgruppe oder eine substituierte oder unsubstituierte Phenylgruppe bedeutet und X eine Alkylengruppe mit 1 bis 6 Kohlenstoffatomen,

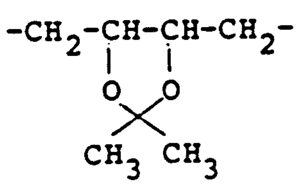 , 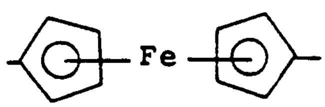 , 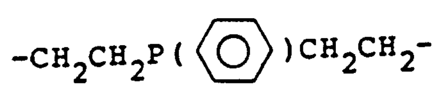

oder eine Binaphtylgruppe bedeutet.

**8.** Verfahren zur Herstellung einer Carbonsäure gemäß Anspruch 7, worin die Palladiumverbindung metallisches Palladium, metallisches Palladium auf einem Feststoffträger, oder ein nullvalenter, divalenter oder tetravalenter Palladiumkomplex ist.

**9.** Verfahren zur Herstellung einer Carbonsäure gemäß Anspruch 8, worin die Palladiumverbindung Palladium-Kohlensstoff, Palladiumchlorid oder Palladiumacetat ist.

**10.** Verfahren zur Herstellung einer Carbonsäure gemäß Anspruch 8, worin die Phosphinverbindung ein Bis(diphenylphosphino)alkan ist.

**11.** Verfahren zur Herstellung einer Carbonsäure gemäß Anspruch 10, worin die Base Tributylamin oder N-Ethylmorpholin ist.

**12.** Verfahren zur Herstellung einer Carbonsäure gemäß Anpruch 11, worin der Kohlenmonoxiddruck 1 bis 200 Atmosphären beträgt.